# EUROPÄISCHE PATENTSCHRIFT

(11) **EP 1 677 818 B1**
(45) Veröffentlichungstag und Bekanntmachung des Hinweises auf die Patenterteilung: **30.11.2011**
(21) Anmeldenummer: 04790683.9
(22) Anmeldetag: 20.10.2004
(51) Int. Cl.: A61K 38/19, A61K 47/12

(54) **STABILE WÄSSRIGE G-CSF-HALTIGE ZUSAMMENSETZUNGEN**
STABLE AQUEOUS G-CSF-CONTAINING COMPOSITIONS
COMPOSITIONS AQUEUSES ET STABLES CONTENANT G-CSF

(30) Priorität: 20.10.2003 DE 10348550
(43) Veröffentlichungstag der Anmeldung: 12.07.2006
(73) Patentinhaber: Hexal AG, 83607 Holzkirchen (DE)
(72) Erfinder: SKUFCA, Peter, 53909 Zülpich (DE); SEIBERT, Fabian, 83629 Weyarn (DE); GRIMM, Rudolf, San Jose, CA 95129 (US)
(74) Vertreter: Winter, Brandl, Fürniss, Hübner, Röss, Kaiser, Polte - Partnerschaft
(86) Internationale Anmeldenummer: PCT/EP2004/011875
(87) Internationale Veröffentlichungsnummer: WO 2005/039620

(56) Entgegenhaltungen:
- EP-A- 0 229 016
- EP-A- 0 373 679
- US-A- 4 675 184
- US-A- 5 078 997
- US-A- 5 151 265
- US-A- 5 350 741
- US-A- 5 597 562
- US-A- 5 919 443
- US-A- 5 919 757
- US-A- 6 162 427
- US-A1- 2003 180 253
- NOMURA H ET AL: "EFFECT OF A DOSING SOLUTION ON THE NASAL ABSORPTION OF NON-GLYCOSYLATED RECOMBINANT HUMAN GRANULOCYTE COLONY-STIMULATING FACTOR IN RATS" BIOLOGICAL & PHARMACEUTICAL BULLETIN (OF JAPAN), PHARMACEUTICAL SOCIETY OF JAPAN, JP, Bd. 19, Nr. 11, 1. November 1996 (1996-11-01), Seiten 1490-1493, XP000636230 ISSN: 0918-6158
- USHIROGAWA Y ET AL: "EFFECT OF ORGANIC ACIDS TRYPSIN INHIBITORS AND DIETARY PROTEIN ON THE PHARMACOLOGICAL ACTIVITY OF RECOMBINANT HUMAN GRANULOCYTE COLONY-STIMULATING FACTOR RHG-CSF IN RATS" INTERNATIONAL JOURNAL OF PHARMACEUTICS (KIDLINGTON), Bd. 81, Nr. 2-3, 1992, Seiten 133-141, XP002319194 ISSN: 0378-5173

## Beschreibung

Gegenstand der vorliegenden Erfindung sind wässrige Zusammensetzungen, die G-CSF enthalten, G-CSF-Lyophilisate oder -Pulver, sowie Arzneimittel-Kits, die diese Lyophilisate oder Pulver enthalten.

G-CSF (Granulocyte-Colony Stimulating Factor; Granulozyten-Kolonien stimulierender Faktor) ist ein natürlich vorkommender Wachstumsfaktor, der zur Familie der Zytokine gehört. G-CSF spielt eine entscheidende Rolle bei der Hämatopoese und fördert Reifung, Proliferation, Differenzierung und Überleben von Neutrophilen und neutrophilen Nachkommenzellen. G-CSF wird klinisch hauptsächlich bei der Tumorbekämpfung und insbesondere zur Behandlung von Neutropenie als Folge von Chemotherapie eingesetzt und findet ferner auch bei Knochenmarkstransplantationen und bei der Behandlung von Infektionskrankheiten Verwendung.

Humanes G-CSF in seiner natürlich vorkommenden Form ist ein Glykoprotein mit einem Molekulargewicht von etwa 20 000 und weist fünf Cystein-Reste auf. Vier dieser Reste bilden zwei intramolekufare Disulfidbrücken, die für die Aktivität des Proteins von wesentlicher Bedeutung sind. Da G-CSF aus seinen natürlichen Quellen nur in geringen Mengen erhältlich ist, werden zur Herstellung von Arzneimitteln hauptsächlich rekombinante Formen von G-CSF verwendet, die beispielsweise durch Expression in Säugerzellen wie CHO (Chinese Hamster Ovary)-Zellen oder Prokaryontenzellen wie E. Coli erhalten werden können. Die in Säugerzellen exprimierten rekombinanten Proteine unterscheiden sich von natürlich vorkommendem G-CSF durch das unterschiedliche Glykosylierungsmuster, während bei den in E. Coli exprimierten Proteinen, die als Folge der bakteriellen Expression einen zusätzlichen N-terminalen Methioninrest aufweisen können, die Glykosylierung vollständig fehlt.

Formulierungen von G-CSF sind aufgrund der hohen Hydrophobizität des Proteins relativ instabil, was besonders für die nicht-glykosylierten rekombinanten Formen des Proteins gilt. Da das Molekül leicht an die Wand von Ampullen, Spritzen o.ä. adsorbiert, Dimere oder höhere Aggregate bildet und chemischen Veränderungen wie Deamidierung, Oxidation, Spaltung von Disulfidbrücken oder Proteolyse unterliegt, kommt es so insbesondere bei längerer Lagerung des Proteins häufig zu Aktivitätsverlusten. Dies ist einerseits aus Kostengründen und andererseits aus therapeutischen Gründen nachteilig, beispielsweise wenn das G-CSF über einen längeren Zeitraum in konstanter Dosierung eingesetzt werden soll. Ferner können die beispielsweise durch Dimerisierung, Oxidation oder Abbau entstandenen Produkte zu möglicherweise unerwünschten Immunreaktionen führen. Herkömmliche G-CSF-Formulierungen sind ferner empfindlich gegenüber mechanischem Stress, wie er beispielsweise durch Schütteln beim Transport der flüssigen Formulierungen auftreten kann, und gegenüber ein- oder mehrmaligem Einfrieren und Auftauen. Beides kann ebenfalls zu einer unerwünschten Bildung von Multimeren und Aggregaten sowie zu Aktivitätsverlust führen.

Die DE-A-37 23 781 beschreibt Arzneimittel mit G-CSF als Wirkstoff, die zur Stabilisierung des Wirkstoffs mindestens ein pharmazeutisch verträgliches grenzflächenaktives Mittel, Saccharid, Protein oder eine pharmazeutisch verträgliche hochmolekulare Verbindung enthalten. Als besonders vorteilhafte grenzflächenaktive Mittel werden Polyoxyethylensorbitanester von aliphatischen Fettsäuren, beispielsweise das Monooleat oder das Monolaureat, vorgeschlagen, die zusammen mit Humanserumalbumin und Mannit eingesetzt werden. Die grenzflächenaktiven Mittel werden bevorzugt in einer Menge von 1 bis 10000 Gewichtsteilen pro Gewichtsteil G-CSF eingesetzt. Die wässrigen phosphatgepufferten Formulierungen, für die ein pH-Wert von 7,4 angegeben wird, sind bei 4°C über längere Zeit stabil.

Die beschriebenen pharmazeutischen Formulierungen haben jedoch einige Nachteile. So ist die Anwesenheit von Tensiden wie Polyoxyethylensorbitanmonooleat (Tween^{®} 80) besonders bei höheren Konzentrationen medizinisch nicht unbedenklich, da es bei der Verabreichung des Arzneimittels zu lokalen Irritationen kommen kann. Ferner wurde beschrieben, dass solche Tenside in den beschriebenen Phosphatpuffern bei höheren Temperaturen wegen der besseren Zugänglichkeit des freien Cysteinrestes von G-CSF die unerwünschte Bildung von Dimeren und Multimeren fördern, so daß die Aktivität von G-CSF bei höheren Temperaturen sehr schnell abnimmt. Die zusätzlich in großen Mengen als Stabilisatoren eingesetzten Proteine und Peptide menschlichen und tierischen Ursprungs bergen ebenfalls ein Risikopotential, da sie wegen ihrer antigenen Eigenschaften immunologische Reaktionen beim Menschen hervorrufen können und auch virale Verunreinigungen nicht vollständig auszuschließen sind.

Die EP-A-0 373 679 offenbart, dass G-CSF über längere Zeit stabil gehalten werden kann, wenn es in Lösungen mit einem pH-Wert von 2,75 bis 4,0 formuliert wird, die vorteilhaft eine möglichst geringe Leitfähigkeit aufweisen. Um die Aggregation von G-CSF zu vermeiden, wird in diesen Formulierungen bevorzugt kein Puffer verwendet, in geringen Mengen von weniger als 2mM können jedoch Carbonsäuren, Citronensäure, Milchsäure oder Weinsäure als Puffersubstanzen eingesetzt werden. Stabile Formulierungen mit pH-Werten nahe dem physiologischen pH-Wert sind jedoch unter diesen Bedingungen nicht möglich.

Herman, A.C. et al. ("Characterisation, Formulation, and Stability of Neupogen® (Filgrastim), a Recombinant Human Granulocyte-Colony Stimulating Factor." In: Formulation Characterisation and Stability of Protein Drugs, S. 303-328, R. Pearlman und Y.J. Wang, Hrsg., Plenum Press, New York, 1996) beschreiben stabilisierte Zusammensetzungen von nichtglykosyliertem rekombinantem G-CSF, die 10 mM Natriumacetat, pH 4,0, 5% Mannit und 0,004% Polysorbat-80 enthalten. Derartige Zusammensetzungen sind bei 2-8°C mehr als 24 Monate stabil. Stabile Zusammensetzungen mit höheren pH-Werten sind jedoch mit diesem System nicht möglich.

Die WO-A-94/14466 offenbart G-CSF-haltige wässrige pharmazeutische Zubereitungen, die Essigsäure, Milchsäure, Citronensäure, Maleinsäure, Phosphorsäure, Arginin und Salze davon als Puffersubstanzen enthalten können und pH-Werte zwischen 2,5 und 5,0 und zwischen 7 und 8 aufweisen. Durch diese Zubereitungen wird die Bildung von Multimeren und Aggregaten von G-CSF bei mechanischen Belastungen, wie sie beispielsweise beim Schütteln von Lösungen auftreten, reduziert. Insbesondere bei höheren Temperaturen nimmt die Aktivität von G-CSF in diesen Zubereitungen jedoch rasch ab und die Langzeitstabilität ist nicht zufrieden stellend.

Die EP-A-0 306 824 beschreibt stabilisierte Präparate von Humanproteinen, insbesondere Erythropoietin, bei denen die Stabilisierung durch Zugabe von Harnstoff, Aminosäuren und Detergens erfolgt. In den für Injektionslösungen verwendeten Puffern, beispielsweise Phosphatpuffern, ist G-CSF bei höheren Temperaturen jedoch nicht stabil genug.

Die WO-A-94/14465 offenbart lyophilisierte pharmazeutische Zubereitungen von G-CSF, die Maltose, Saccharose, Raffinose, Trehalose oder Aminozucker enthalten. Die wässrigen Lösungen dieser Lyophilisate sind jedoch über längere Zeiträume ebenfalls nicht hinreichend stabil.

Die EP-A-1 197 221 offenbart langzeitstabile G-CSF-Formulierungen mit pH-Werten zwischen 5 und 7, die ein oder mehrere Aminosäuren aus der Gruppe Lysin, Histidin, Arginin, Asparaginsäure, Glutaminsäure, Threonin und Asparagin sowie ein oder mehrerer hydrophobe Aminosäuren enthalten. Zur Vermeidung der Oxidation von Methioninresten im G-CSF-Molekül wird der Formulierung die Aminosäure Methionin zugesetzt.

Aufgabe der vorliegenden Erfindung war es, wässrige G-CSF-haltige Zusammensetzungen bereitzustellen, die auch in Abwesenheit von Serumproteinen über einen breiten pH-Bereich und bei höheren Temperaturen über einen längeren Zeitraum stabil sind und sich insbesondere zur pharmazeutischen Anwendung eignen.

Es wurde nun überraschend gefunden, dass wässrige G-CSF-haltige Zusammensetzungen, die Succinat als Puffersubstanzen, wenigstens ein Tensid sowie ein isotonisierendes Mittel, ausgewählt aus Mannit und/oder Sorbit, enthalten, auch bei höheren Temperaturen über einen breiten pH-Bereich und sogar bei pH-Werten, die nahe den physiologischen Bedingungen liegen, über lange Zeit stabil sind, da in solchen Zusammensetzungen kaum chemische Veränderungen wie Dimerisierung oder Oxidation des G-CSF-Moleküls auftreten. Daher geht auch bei längerer Lagerung kaum Aktivität verloren. Auch bei der Rekonstitution oder dem Auflösen von G-CSF-haltigen Lyophilisaten oder Pulvern, bei mechanischer Belastung, wie sie beispielsweise beim Filtrieren G-CSF-haltiger Zusammensetzungen, beim Abfüllen in Ampullen, beim Aufziehen in Spritzen und beim Transport auftreten kann, und beim Einfrieren und Auftauen verhindert die Anwesenheit von Succinat und den weiteren Inhaltsstoffen unerwünschte Aggregatbildung oder andere Nebenreaktionen des G-CSF-Proteins und damit Aktivitätsverluste.

Gegenstand der vorliegenden Erfindung sind demnach wässrige G-CSF-haltige Zusammensetzungen gemäß Anspruch 1, Verfahren zu ihrer Herstellung und ihre Verwendung zur Herstellung pharmazeutischer Präparate.

Ein weiterer Gegenstand der vorliegende Erfindung sind Lyophilisate und Pulver gemäß Anspruch 10, Verfahren zu ihrer Herstellung sowie ihre Verwendung zur Herstellung pharmazeutischer Präparate.

Weitere Ausführungsformen der Erfindung ergeben sich aus den Ansprüchen und der nachfolgenden Beschreibung.
Fig. 1 zeigt den Restgehalt von monomerem G-CSF nach 4 und 8 Wochen Inkubation bei 25°C in 20 mM Succinatpuffer bei pH-Werten zwischen 4,5 und 6,0 im Vergleich mit 10 mM Acetatpuffer bei pH 4,0, bestimmt durch RP-HPLC und ausgedrückt als % Peakfläche (PA) des Anfangsgehalts an monomerem G-CSF (100 %) an Tag 0.
Fig. 2 zeigt den Restgehalt von monomerem G-CSF nach 4 und 8 Wochen Inkubation bei 25°C in 10 mM Succinatpuffer bei pH-Werten zwischen 4,0 und 6,0 im Vergleich mit 10 mM Acetatpuffer bei pH 4,0.
Fig. 3 zeigt den Restgehalt von monomerem G-CSF nach 4 und 8 Wochen Inkubation bei 25°C in 5 mM Succinatpuffer bei pH-Werten zwischen 4,0 und 6,0 im Vergleich mit 10 mM Acetatpuffer bei pH 4,0.
Fig. 4 zeigt den Restgehalt von monomerem G-CSF nach 4 und 8 Wochen Inkubation bei 25°C in 20 mM Tartratpuffer bei pH-Werten zwischen 4,0 und 6,0 im Vergleich mit 10 mM Acetatpuffer bei pH 4,0.
Fig. 5 zeigt den Restgehalt von monomerem G-CSF nach 4 und 8 Wochen Inkubation bei 25°C in 10 mM Tartratpuffer bei pH-Werten zwischen 4,5 und 6,0 im Vergleich mit 10 mM Acetatpuffer bei pH 4,0.
Fig. 6 zeigt den Restgehalt von monomerem G-CSF nach 4 und 8 Wochen Inkubation bei 25°C in 5 mM Tartratpuffer bei pH-Werten zwischen 4,0 und 6,0 im Vergleich mit 10 mM Acetatpuffer bei pH 4,0.
Fig. 7 zeigt den Restgehalt von monomerem G-CSF nach 13 und 20 Tagen Inkubation bei 37°C in 10 mM Phosphatpuffer bei pH 4,5, 5,0 und 7,5.
Fig. 8 zeigt den Restgehalt von monomerem G-CSF nach 30 und 90 Tagen Inkubation bei 37°C in 10 mM Succinat- oder Tartratpuffer bei pH 5,0 im Vergleich mit 10 mM Acetatpuffer bei pH 4,0.

Das G-CSF-Protein in den erfindungsgemäßen Zusammensetzungen kann jedes beliebige G-CSF-Protein von Säugern, insbesondere Menschen, oder eine davon abgeleitete Variante sein, sofern diese Variante im Wesentlichen die für humanen G-CSF charakteristische biologische Aktivität bei der Hämatopoese besitzt. Der Begriff G-CSF, wie er hier verwendet wird, umfasst demnach sowohl G-CSF natürlicher Herkunft als auch synthetisch oder rekombinant hergestellten G-CSF sowie Varianten davon, beispielsweise rekombinantes humane Proteine mit einem N-terminalen Methioninrest, wie sie bei der Expression des G-CSF-Gens in Prokaryonten erhalten werden, Fusionsproteine von G-CSF, sowie G-CSF-Proteine, wie sie die durch Austausch, Deletion oder Insertion von ein oder mehreren Aminosäuren des natürlich vorkommenden G-CSF erhalten werden. G-CSF kann glykosyliert oder nicht-glykosyliert sein. Nicht-glykosylierter G-CSF wird beispielsweise bei der Expression in Prokaryontenzellen wie E. Coli erhalten, während glykosylierter G-CSF entweder bei der Isolierung aus natürlichen Quellen, bei der Expression in Eukaryontenzellen wie CHO-Zellen oder durch synthetische Glykosylierung erhalten werden kann. Synthetisch modifizierter G-CSF kann beispielsweise durch enzymatische Glykosylierung oder auch durch chemische PEGylierung erhalten werden. G-CSF-Varianten, wie sie in den erfindungsgemäßen Zusammensetzungen verwendet werden können, sind beispielsweise in der EP-A-0 456 200 beschrieben. Bevorzugt wird rekombinanter, nicht-glykosylierter G-CSF in den erfindungsgemäßen Zusammensetzungen verwendet, besonders bevorzugt umfaßt der G-CSF die Aminosäuresequenz von humanem G-CSF, wie sie beispielsweise in der DE-A-37 23 781 angegeben ist, oder eine davon abgeleitete Sequenz.

Zur Herstellung der erfindungsgemäßen wässrigen Zusammensetzungen, der Lyophilisate und der Pulver kann die Puffersubstanz Succinat sowohl in Form der freien Säure als auch in Form der Salze eingesetzt werden. Als Salze der Bernsteinsäure werden insbesondere die physiologisch verträglichen Salze eingesetzt, beispielsweise Alkali-, Erdalkali- oder Ammoniumalze. Bevorzugt sind die Alkali- und Ammoniumsalze, besonders bevorzugt die Di-NatriumSalze.

Die Konzentration der Puffersubstanz Succinat liegt bei 0,5 bis 100 mM, um Aggregatbildungen zu vermeiden. Vorzugsweise liegen die Konzentrationen, in denen die Puffersubstanz in der wässrigen Zusammensetzung eingesetzt wird, zwischen 1 und 100 mM und ganz besonders bevorzugt zwischen 1 und 50 mM, beispielsweise zwischen 2 und 20 mM.

Der pH-Wert der erfindungsgemäßen Zusammensetzungen liegt üblicherweise zwischen 3,5 und 6,0, beispielsweise zwischen 4,0 und 5,9. Vorzugsweise ist der pH größer 4,0 und liegt beispielsweise zwischen 4,1 und 5,7, insbesondere zwischen 4,2 und 5,5, beispielsweise zwischen 4,5 und 5,5. Falls gewünscht kann der pH-Wert auch noch mit Hilfe anderer Säuren oder Basen auf den gewünschten Wert eingestellt werden. Geeignete Säuren sind beispielsweise Salzsäure, Phosphorsäure, Essigsäure, Citronensäure und Natrium- oder Kaliumdihydrogenphosphat. Geeignete Basen sind beispielsweise Alkali- und Erdalkalihydroxide, Alkalicarbonate, Alkaliacetate, Alkalicitrate und Di-Alkalihydrogenphospate, beispielsweise Natriumhydroxid, Natriumacetat, Natriumcarbonat, Natriumcitrat, Di-Natrium- und Di-Kaliumhydrogenphosphat sowie Ammoniak.

Die Konzentration von G-CSF in den erfindungsgemäßen Zusammensetzungen ist im Wesentlichen abhängig vom Verwendungszweck. Die obere Grenzkonzentration ergibt sich aus der Löslichkeit von G-CSF im Puffer. In pharmazeutischen Zusammensetzungen liegt G-CSF in einer pharmazeutisch wirksamen Menge vor, wobei die Konzentration gewöhnlich nicht mehr als 5mg/ml beträgt und beispielsweise zwischen 0,0001 und 5 mg/ml, vorzugsweise zwischen 0,0005 und 4 mg/ml und besonders bevorzugt zwischen 0,001 und 2,5 mg/ml, beispielsweise zwischen 0,01 und 1,5 mg/ml, liegt. In Bulklösungen (höher konzentrierten Ausgangslösungen) kann die Konzentration jedoch auch ohne weiteres 10 mg/ml und mehr annehmen.

Die erfindungsgemäßen Zusammensetzungen enthalten weiter wenigstens ein Tensid um ein isotonisierendes Mittel, ausgewählt aus Mannit und/oder Sorbit.

Bevorzugt enthält die erfindungsgemäße Zusammensetzung ein oder mehrere Tenside, beispielsweise nichtionische Tenside, wie sie in der EP-A-1 197 221 beschrieben sind, insbesondere Polyoxyethylensorbitanester aliphatischer Fettsäuren. Hier sind beispielsweise Polyoxyethylensorbitanmonolaurat (erhältlich unter dem Handelsnamen Polysorbat 20), Polyoxyethylensorbitanmonopalmitat (Polysorbat 40), Polyoxyethylensorbitanmonostearat (Polysorbat 60), Polyoxyethylensorbitantristearat (Polysorbat 65), Polyoxyethylensorbitanmonooleat (Polysorbat 80) und Polyoxyethylensorbitantrioleat (Polysorbat 85) zu nennen, wobei Polyoxyethylensorbitanmonopalmitat und Polyoxyethylensorbitanmonooleat bevorzugt sind. Diese Tenside können, falls gewünscht, aufgrund des vorteilhaften stabilisierenden Puffersystems der Erfindung in sehr geringen Mengen eingesetzt werden, beispielsweise in Mengen von 0,0005 bis 0,04 % (w/v), vorzugsweise von 0,001 bis 0,02 % (w/v), bezogen auf das Gesamtvolumen der Zusammensetzung.

Vorteilhaft sind die erfindungsgemäßen Zusammensetzungen, insbesondere wenn sie für pharmazeutische Zwecke eingesetzt werden, mit dem Blut des Patienten isotonisch. Dies kann bereits durch die Wahl geeigneter Konzentrationen an Puffersubstanzen erfolgen. Zur Herstellung physiologisch gut verträglicher Zusammensetzungen werden die isotonisierenden Mittel Mannit und/oder Sorbit zugesetzt.

Isotonisierende Mittel werden zweckmäßig in Mengen von bis zu 10,0 % (w/v) zugesetzt, bezogen auf das Gesamtvolumen der Zusammensetzung. Bevorzugt werden Mengen von bis zu 7,5 %, besonders bevorzugt bis zu 6,0 %, beispielsweise zwischen 0,1 und 5,5 % (w/v) eingesetzt.

Die Herstellung der erfindungsgemäßen Zusammensetzungen kann in an sich bekannter Weise erfolgen. Üblicherweise werden die Puffersubstanzen und die Tenside und isotonisierenden Mittel in den geeigneten Mengen in dem wässrigen Lösungsmittel, gewöhnlich sterilem Wasser, gelöst. Falls erforderlich wird der pH-Wert mit Succinat oder mit anderen Säuren oder Basen, wie den oben beispielhaft genannten, eingestellt. Nach einem üblichen Sterilisierungsschritt, beispielsweise Filtration durch ein Sterilfilter, wird G-CSF in den gewünschten Konzentrationen zugegeben. Es ist aber auch ohne weiteres möglich, G-CSF in einer wässrigen Lösung vorzulegen und den pH anschließend mit Succinat auf den gewünschten Wert einzustellen.

Die erfindungsgemäßen Zusammensetzungen finden insbesondere als pharmazeutische Zusammensetzungen Verwendung, wobei die gegebenenfalls vorhandenen Stabilisierungsmittel und die Hilfs- und Zusatzstoffe physiologisch verträglich sein müssen. Die pharmazeutischen Zusammensetzungen können in den verschiedenartigsten Applikationsformen eingesetzt werden. Beispielsweise können die Zusammensetzungen als Injektions- oder Infusionslösungen, insbesondere zur intravenösen, intramuskulären oder subkutanen Verabreichung, oder als Zusammensetzungen zur oralen Verabreichung vorliegen. Die Zusammensetzungen können jedoch auch zur Herstellung weiterer pharmazeutischer Applikationsformen verwendet werden, beispielsweise von Hydrogelen oder Liposomen. Solche pharmazeutischen Zubereitungen können auf sämtlichen Indikationsgebieten eingesetzt werden, auf denen G-CSF zur Anwendung kommen kann, beispielsweise zur Behandlung von Neutropenie, bei Knochenmarkstransplantationen und bei der Behandlung von Infektionskrankheiten und Tumorerkrankungen.

Gegenstand der vorliegenden Erfindung sind ferner G-CSF-haltige Lyophilisate und Pulver, die Succinat in Form der freien Säure und/oder eines Salzes davon, umfassen. Solche Lyophilisate und Pulver lassen sich beispielsweise in an sich bekannter und einfacher Weise durch Lyophilisieren oder z.B. durch Sprühtrocknung aus den zuvor beschriebenen wässrigen Zusammensetzungen erhalten. In diesen Lyophilisaten und Pulvern liegen G-CSF, Succinat sowie die Tenside und isotonisierenden Mittel in solchen Mengen vor, dass nach erneutem Auflösen in Wasser G-CSF-haltige Zusammensetzungen erhalten werden, die wie die entsprechenden wässrigen Zusammensetzungen auch bei höheren Temperaturen über einen längeren Zeitraum stabil sind.

Die erfindungsgemäßen Lyophilisate oder Pulver können beispielsweise in Form eines Arzneimittel-Kits bereitgestellt werden, in dem Lyophilisat oder Pulver räumlich getrennt von einer geeigneten Menge eines wässrigen Lösungsmittels vorliegen. Die stabile gepufferte wässrige Zusammensetzung kann dann zu jedem gewünschten Zeitpunkt, beispielsweise vom medizinischen Personal, hergestellt werden.

Die vorliegende Erfindung wird nun anhand der folgenden Beispiele näher erläutert, ohne dass in diesen Beispielen eine Beschränkung zu sehen ist.

### Beispiele

### 1. Stabilität G-CSF-haltiger Zusammensetzungen nach Langzeitinkubation und bei erhöhtem pH und erhöhter Temperatur

G-CSF-haltige Zusammensetzungen wurden bei Raumtemperatur hergestellt, indem die Puffersubstanzen Succinat und Tartrat in Form der Dinatriumsalze zusammen mit Polysorbat-80 und Mannit zunächst in destilliertem und sterilem Wasser gelöst wurden und anschließend der pH-Wert mit Succinat- oder Tartratpuffer auf den gewünschten Wert eingestellt wurde. Im Handel erhältlicher, nicht-glykosylierter rekombinanter humaner G-CSF wurde nach Filtration durch ein Sterilfilter (Porengröße 0,2 µm, Millipore^{®} zugegeben.

Die genauen Formulierungen der hergestellten erfindungsgemäßen Zusammensetzungen und deren pH-Werte sind in den nachfolgenden Tabellen 1 bis 6 angegeben.

**Tabelle 1: G-CSF-haltige Formulierung mit 20 mM Succinatpuffer**

| | **Formulierung** | | | | | | | | |
|---|---|---|---|---|---|---|---|---|---|
| | **1** | **2** | **3** | **4** | **5** | **6** | **7** | **8** | **9** |
| Substanz | | | | | | | | | |
| G-CSF [mg/ml] | 0,3 | 0,3 | 0,3 | 0,3 | 0,3 | 0,3 | 0,3 | 0,3 | 0,3 |
| Mannit [%, w/v] | 0,5 | 0,5 | 0,5 | 0,5 | 0,5 | 0,5 | 0,5 | 0,5 | 0,5 |
| Polysorbat-80 [%, w/v] | 0,004 | 0,004 | 0,004 | 0,004 | 0,004 | 0,004 | 0,004 | 0,004 | 0,004 |
| **Succinat-puffer [mM]** | **20** | **20** | **20** | **20** | **20** | **20** | **20** | **20** | **20** |
| pH | 4,00 | 4,25 | 4,50 | 4,75 | 5,00 | 5,25 | 5,50 | 5,75 | 6,00 |

**Tabelle 2: G-CSF-haltige Formulierung mit 10 mM Succinatpuffer**

| | **Formulierung** | | | | | | | | |
|---|---|---|---|---|---|---|---|---|---|
| | **10** | **11** | **12** | **13** | **14** | **15** | **16** | **17** | **18** |
| Substanz | | | | | | | | | |
| G-CSF [mg/ml] | 0,3 | 0,3 | 0,3 | 0,3 | 0,3 | 0,3 | 0,3 | 0,3 | 0,3 |
| Mannit [%, w/v] | 0,5 | 0,5 | 0,5 | 0,5 | 0,5 | 0,5 | 0,5 | 0,5 | 0,5 |
| Polysorbat-80 [%, w/v] | 0,004 | 0,004 | 0,004 | 0,004 | 0,004 | 0,004 | 0,004 | 0,004 | 0,004 |
| **Succinat-puffer [mM]** | **10** | **10** | **10** | **10** | **10** | **10** | **10** | **10** | **10** |
| pH | 4,00 | 4,25 | 4,50 | 4,75 | 5,00 | 5,25 | 5,50 | 5,75 | 6,00 |

**Tabelle 3: G-CSF-haltige Formulierung mit 5 mM Succinatpuffer**

| | **Formulierung** | | | | | | | | |
|---|---|---|---|---|---|---|---|---|---|
| | **19** | **20** | **21** | **22** | **23** | **24** | **25** | **26** | **27** |
| Substanz | | | | | | | | | |
| G-CSF [mg/ml] | 0,3 | 0,3 | 0,3 | 0,3 | 0,3 | 0,3 | 0,3 | 0,3 | 0,3 |
| Mannit [%, w/v] | 0,5 | 0,5 | 0,5 | 0,5 | 0,5 | 0,5 | 0,5 | 0,5 | 0,5 |
| Polysorbat-80 [%, w/v] | 0,004 | 0,004 | 0,004 | 0,004 | 0.004 | 0,004 | 0,004 | 0,004 | 0,004 |
| **Succinat-puffer [mM]** | **5** | **5** | **5** | **5** | **5** | **5** | **5** | **5** | **5** |
| pH | 4,00 | 4,25 | 4,50 | 4,75 | 5,00 | 5,25 | 5,50 | 5,75 | 6,00 |

**Tabelle 4: G-CSF-haltige Formulierung mit 20 mM Tartratpuffer**

| | **Formulierung** | | | | | | | | |
|---|---|---|---|---|---|---|---|---|---|
| | **28** | **29** | **30** | **31** | **32** | **33** | **34** | **35** | **36** |
| Substanz | | | | | | | | | |
| G-CSF [mg/ml] | 0,3 | 0,3 | 0,3 | 0,3 | 0,3 | 0,3 | 0,3 | 0,3 | 0,3 |
| Mannit [%, w/v] | 0,5 | 0,5 | 0,5 | 0,5 | 05 | 0,5 | 0,5 | 0,5 | 0,5 |
| Polysorbat-80 [%, w/v] | 0,004 | 0,004 | 0,004 | 0,004 | 0,004 | 0,004 | 0,004 | 0,004 | 0,004 |
| **Tartratpuffer [mM]** | **20** | **20** | **20** | **20** | **20** | **20** | **20** | **20** | **20** |
| pH | 4,00 | 4,25 | 4,50 | 4,75 | 5,00 | 5,25 | 5,50 | 5,75 | 6,00 |

**Tabelle 5: G-CSF-haltige Formulierung mit 10 mM Tartratpuffer**

| | **Formulierung** | | | | | | | | |
|---|---|---|---|---|---|---|---|---|---|
| | **37** | **38** | **39** | **40** | **41** | **42** | **43** | **44** | **45** |
| Substanz | | | | | | | | | |
| G-CSF [mg/ml] | 0,3 | 0,3 | 0,3 | 0,3 | 0,3 | 0,3 | 0,3 | 0,3 | 0,3 |
| Mannit [%, w/v] | 0,5 | 0,5 | 0,5 | 0,5 | 0,5 | 0,5 | 0,5 | 0,5 | 0,5 |
| **Polysorbat-80 [%, w/v]** | **0,004** | **0,004** | **0,004** | **0,004** | **0,004** | **0,004** | **0,004** | **0,004** | **0,004** |
| Tartratpuffer [mM] | 10 | 10 | 10 | 10 | 10 | 10 | 10 | 10 | 10 |
| pH | 4,00 | 4,25 | 4,50 | 4,75 | 5,00 | 5,25 | 5,50 | 5,75 | 6,00 |

**Tabelle 6: G-CSF-haltige Formulierung mit 5 mM Tartratpuffer**

| | **Formulierung** | | | | | | | | |
|---|---|---|---|---|---|---|---|---|---|
| | **46** | **47** | **48** | **49** | **50** | **51** | **52** | **53** | **54** |
| Substanz | | | | | | | | | |
| G-CSF [mg/ml] | 0,3 | 0,3 | 0,3 | 0,3 | 0,3 | 0,3 | 0,3 | 0,3 | 0,3 |
| Mannit [%, w/v] | 0,5 | 0,5 | 0,5 | 0,5 | 0,5 | 0,5 | 0,5 | 0,5 | 0,5 |
| Polysorbat-80 [%, w/v] | 0,004 | 0,004 | 0,004 | 0,004 | 0,004 | 0.004 | 0,004 | 0,004 | 0,004 |
| **Tartratpuffer [mM]** | **5** | **5** | **5** | **5** | **5** | **5** | **5** | **5** | **5** |
| pH | 4,00 | 4,25 | 4,50 | 4,75 | 5,00 | 5,25 | 5,50 | 5,75 | 6,00 |

500 µl der hergestellten Zusammensetzungen wurden in Eppendorfröhrchen bei 4 ± 1°C, 25 ± 1°C und 37 ± 1°C 8 Wochen lang inkubiert. Als Vergleich dienten eine G-CSF-haltige Formulierung mit 0,3 mg/ml G-CSF in 10 mM Natriumacetatpuffer, pH 4,0, mit 0,5% (w/v) Mannit und 0,004 % (w/v) Polysorbat-80, sowie eine Formulierung mit 0,3 mg/ml G-CSF in 10 mM Phosphatpuffer, pH 4,0, 5,0 und 7,5, mit 0,5% (w/v) Mannit und 0,004 % (w/v) Polysorbat-80.

Während der Inkubation wurden zu verschiedenen Zeitpunkten Proben genommen und hinsichtlich ihres Restgehalts an chemisch unmodifiziertem monomerem G-CSF und hinsichtlich der biologischen Aktivität von G-CSF analysiert. Ein hoher Restgehalt an monomerem G-CSF und/oder eine im Wesentlichen gleich bleibende biologische Aktivität des G-CSF-Proteins zeigen an, dass an dem Protein keine oder nur geringe chemische Veränderungen erfolgt sind.

Die Bestimmung des Restgehalts von chemisch unmodifiziertem monomerem G-CSF erfolgte mittels Reverse Phase-Hochleistungschromatographie (RP-HPLC) mit einer C4 Vydac-Säule. Die mobile Phase enthielt mit Trifluoressigsäure (TFA) angesäuertes Wasser als Eluens A und mit TFA angesäuertes Acetonitril als Eluens B. Die Chromatographie wurde 1 Stunde bei einer Durchflussgeschwindigkeit von 0,2 ml/min mit einem linearen Gradienten von A und B durchgeführt. Das Einspritzvolumen betrug 5 µl. Die Detektionswellenlänge war 206 nm und die Auswertung erfolgte mit einer bekannten G-CSF-Verdünnung als externem Standard. Der Restgehalt an G-CSF wurde entsprechend der Methode von Herman, A.C. (a.a.O.) als % Peakfläche (PA) des Anfangsgehalts an monomerem G-CSF an Tag 0 bestimmt, die als 100 % definiert wurde.

Der Nachweis der Stabilisierung von G-CSF durch die Bestimmung der biologischen Aktivität von G-CSF nach Langzeitinkubation erfolgte mittels eines NFS-60-Bioassays (Tohyama, K. et al., Japanese J. Cancer Res. 80:335-340, 1989). Hierbei wurde die G-CSF-Aktivität festgestellt, indem die Induktion der Zellproliferation als Reaktion auf verschiedene Konzentrationen von G-CSF gemessen wurde. Die Proliferation der NFS-60-Zellen wurde durch Bestimmung der Dehydrogenase-Aktivität verfolgt. Dehydrogenase reduziert 3-(4,5-Dimethylthiatholyl-2)-2,5-diphenyltetrazoliumbromid (MTT) zu Formazan, welches photometrisch bei einer Detektionswellenlänge von 570 nm mit einer Referenzwellenlänge von 620 nm gemessen werden kann. Die Dehydrogenase-Aktivität und damit die Menge gebildetes Formazan korrelieren direkt mit der Zellzahl der NFS-60-Zellen.

Die erhaltenen Ergebnisse werden nachfolgend beschrieben.

### Restgehalt von G-CSF nach Inkubation bei 25°C in Formulierungen mit Succinatpuffer

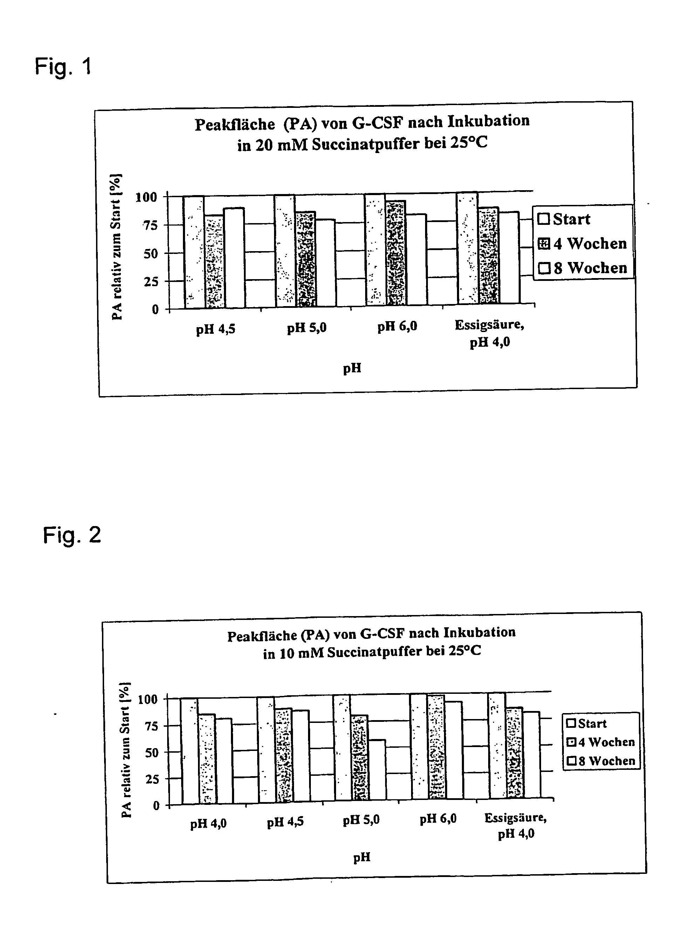

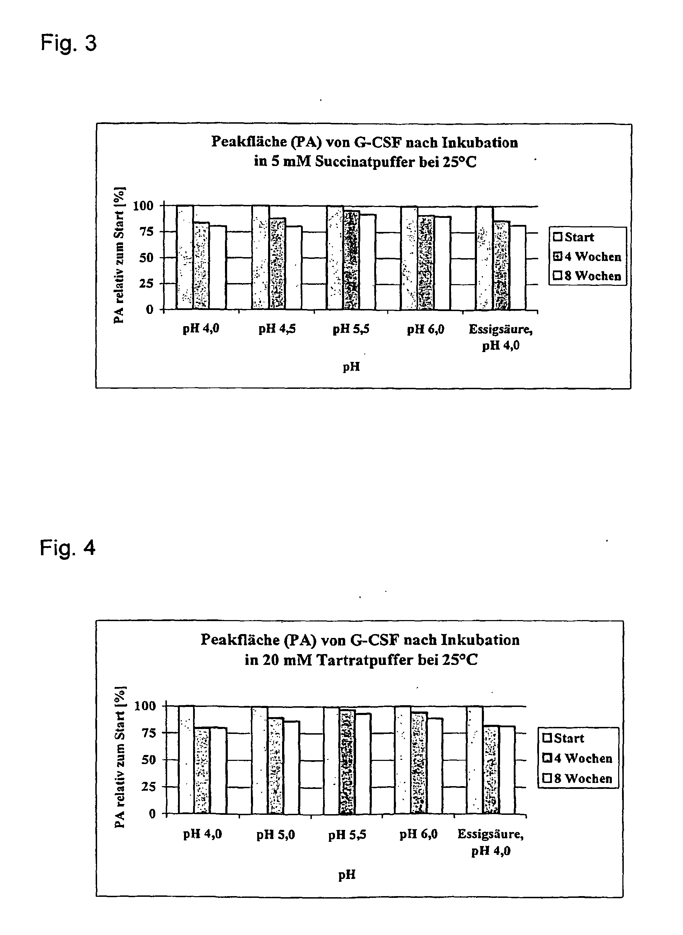

Die Analyse der bei 25°C inkubierten Zusammensetzungen 1 bis 27 (Tabelle 1-3; Fig. 1-3) mittels RP-HPLC nach 4 und 8 Wochen Inkubation zeigte, dass der Gehalt an G-CSF verglichen mit dem Anfangsgehalt der Zusammensetzungen in 20, 10 und 5 mM Succinatpuffer auch noch nach 8 Wochen Inkubation bei 25°C sehr hoch war. Die Stabilität von G-CSF in den erfindungsgemäßen Zusammensetzungen ist vergleichbar mit der Stabilität von G-CSF in einer herkömmlichen Formulierung mit 10 mM Acetat, pH 4,0, jedoch bei deutlich höheren pH-Werten. Die Figuren 1 bis 3 zeigen repräsentative Beispiele für Formulierungen mit Succinatpuffer bei verschiedenen pH-Werten im Vergleich mit einer herkömmlichen Acetat-Formulierung bei pH 4,0. Figur 1 zeigt den Gehalt von G-CSF zu Beginn des Experiments (100 %) und nach 4 und 8 Wochen Inkubation in 20 mM Succinatpuffer bei pH 4,5, 5,0 und 6,0. Figur 2 zeigt den Gehalt von G-CSF zu Beginn des Experiments (100 %) und nach 4 und 8 Wochen Inkubation in 10 mM Succinatpuffer bei pH 4,0, 4,5, 5,0 und 6,0. Figur 3 zeigt den Gehalt von G-CSF zu Beginn des Experiments und nach 4 und 8 Wochen Inkubation in 5 mM Succinatpuffer bei pH 4,0, 4,5, 5,5 und 6,0.

Bei Inkubation bei 4°C verhielten sich acetat- und succinat-/tartratgepufferte G-CSF-Lösungen in den beschriebenen pH-Bereichen vergleichbar (Werte nicht gezeigt).

### Vergleichsbeispiel

### Restgehalt von G-CSF nach Inkubation bei 25°C in Formulierungen mit Tartratpuffer

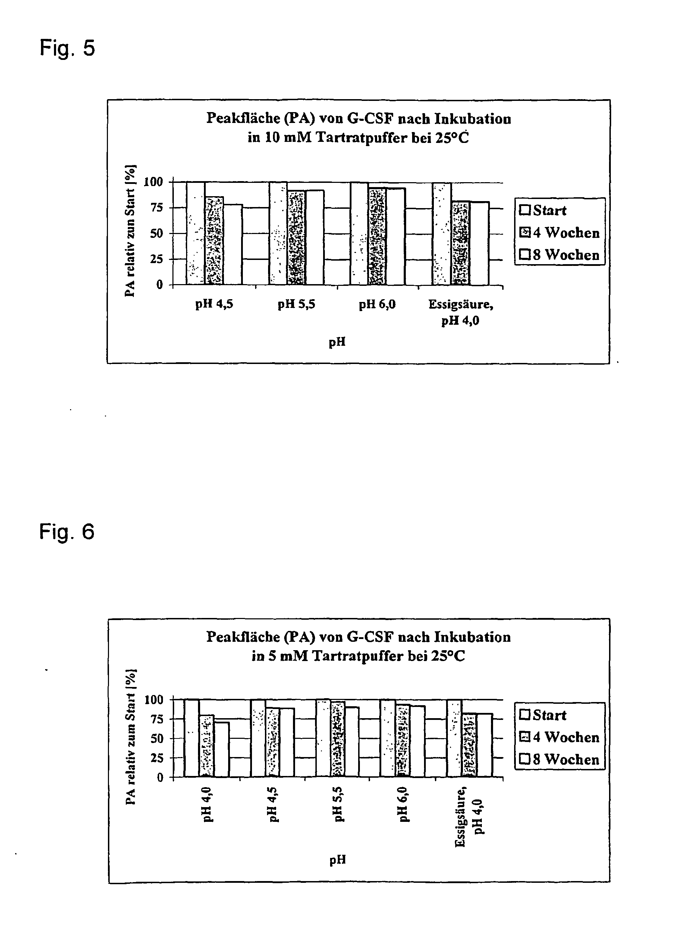

Die Analyse der bei 25°C inkubierten Zusammensetzungen 28 bis 54 (Tabelle 4-6, Fig. 4-6) mittels RP-HPLC nach 4 und 8 Wochen Inkubation zeigte, dass der Gehalt an G-CSF verglichen mit dem Anfangsgehalt der Zusammensetzungen in 20, 10 und 5 mM Tartratpuffer auch noch nach 8 Wochen Inkubation bei 25°C sehr hoch war. Auch in diesem Fall ist die Stabilität von G-CSF in den erfindungsgemäßen Zusammensetzungen vergleichbar mit der Stabilität von G-CSF in einer herkömmlichen Formulierung mit 10 mM Acetat, pH 4,0. Die Figuren 4 bis 6 zeigen repräsentative Beispiele für Formulierungen mit Tartratpuffer bei verschiedenen pH-Werten im Vergleich mit einer herkömmlichen Acetat-Formulierung bei pH 4,0. Figur 4 zeigt den Gehalt von G-CSF zu Beginn des Experiments (100 %) und nach 4 und 8 Wochen Inkubation in 20 mM Tartratpuffer bei pH 4,0, 5,0, 5,5 und 6,0. Figur 5 zeigt den Gehalt von G-CSF zu Beginn des Experiments (100 %) und nach 4 und 8 Wochen Inkubation in 10 mM Tartratpuffer bei pH 4,5, 5,5 und 6,0. Figur 6 zeigt den Gehalt von G-CSF zu Beginn des Experiments (100 %) und nach 4 und 8 Wochen Inkubation in 5 mM Tartratpuffer bei pH 4,0, 4,5, 5,5 und 6,0. Bei Inkubation bei 4°C verhielten sich acetat- und succinat-/tartratgepufferte G-CSF-Lösungen in den beschriebenen pH-Bereichen vergleichbar (Werte nicht gezeigt).

### Restgehalt von G-CSF nach Inkubation bei 37°C in verschiedenen Puffern

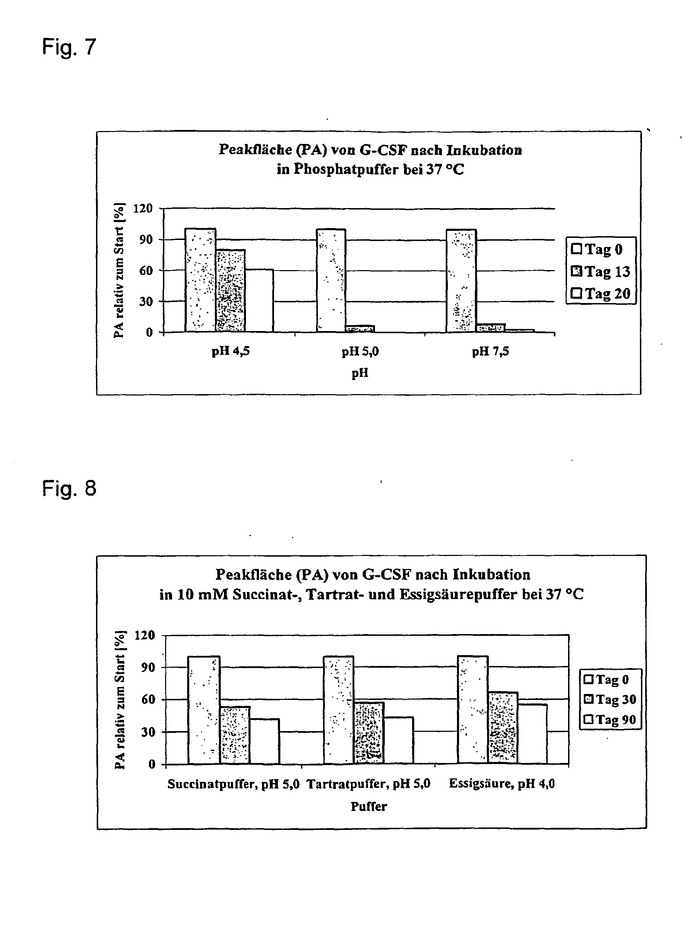

In einem weiteren Versuch wurde die Langzeitstabilität von G-CSF bei 37°C in 10 mM Succinat- und Tartratpuffer, pH 5,0 (Formulierungen 58 und 59), in 10 mM Acetatpuffer, pH 4,0 (Formulierung 60), sowie in 10 mM Phosphatpuffer, pH 4,5, 5,0 und 7,5 (Formulierungen 55-57), bestimmt (Tabelle 7). Der Gehalt von monomerem G-CSF, der wie in den vorhergehenden Versuchen mittels RP-HPLC bestimmt wurde, ist in den Figuren 7 und 8 graphisch dargestellt. Die Ergebnisse zeigen, dass der Gehalt von G-CSF in den Formulierungen mit Succinat- und Tartratpuffer auch noch nach 90 Tagen Inkubation mit dem Gehalt vergleichbar ist, der in 10 mM Acetat, pH 4,0, beobachtet wird. Dagegen nimmt der G-CSF-Gehalt in den Formulierungen mit 10 mM Phosphatpuffer bei pH 5,0 bereits nach 13 Tagen drastisch ab und ist nach 20 Tagen vernachlässigbar gering.

**Tabelle 7: G-CSF-haltige Formulierung in 10 mM Phosphat-, Succinat-, Tartrat- und Acetatpuffer**

| | **Formulierung** | | | | | |
|---|---|---|---|---|---|---|
| | 55 | 56 | 57 | 58 | 59 | 60 |
| Substanz | | | | | | |
| G-CSF [mg/ml] | 0,3 | 0,3 | 0,3 | 0,3 | 0,3 | 0,3 |
| Mannit [%, w/v] | 0,5 | 0,5 | 0,5 | 0,5 | 0,5 | 0,5 |
| Polysorbat-80 [%, w/v] | 0,004 | 0,004 | 0,004 | 0,004 | 0,004 | 0,004 |
| Inkubationsdauer [Tage] | 20 | 20 | 20 | 90 | 90 | 90 |
| **Phosphatpuffer [mM]** | **10** | **10** | **10** | - | - | - |
| **Succinatpuffer [mM]** | - | - | - | **10** | - | - |
| **Tartratpuffer [mM]** | - | - | - | - | **10** | - |
| **Essigsäure [mM]** | - | - | - | - | - | **10** |
| pH | 4,5 | 5,0 | 7,5 | 5,0 | 5,0 | 4,0 |

### Biologische Aktivität von G-CSF nach Inkubation bei 25°C und Bei 37°C in Succinat- und Tartratpuffern

Die Analyse einiger repräsentativer Formulierungen (3, 4, 5, 13, 14, 23, 32 und 41 der Tabellen 1 bis 6), welche bei 25°C und 37°C eingelagert wurden, mit dem weiter oben unter 2.2 beschriebenen Bioassay zeigt, dass die biologische Aktivität von G-CSF-Formulierungen mit den erfindungsgemäßen Puffersubstanzen Succinat oder Tartrat bei verschiedenen Konzentrationen (5 bis 20 mM) und bei verschiedenen pH-Werten (pH 4,5 bis pH 5,5) auch unter Stressbedingungen von 25°C und 37°C noch nach 90 Tagen Inkubation zwischen 80 und 120 % der anfänglichen Aktivität liegt (Tabelle 8).

**Tabelle 8: Biologische Aktivität von G-CSF-Formulierungen**

| | **Formulierung** | | | | | | | | | | | |
|---|---|---|---|---|---|---|---|---|---|---|---|---|
| | **3** | **4** | **5** | **13** | **14** | **23** | **5** | **14** | **32** | **34** | **32** | **41** |
| Inkubations-temperatur [°C] | 25 | 25 | 25 | 25 | 25 | 25 | 37 | 37 | 25 | 25 | 37 | 37 |
| Inkubationsdauer [Tage] | 60 | 60 | 90 | 60 | 90 | 90 | 40 | 90 | 70 | 70 | 30 | 90 |
| **Succinatpuffer [mM]** | **20** | **20** | **20** | **10** | **10** | **5** | **20** | **10** | - | - | - | - |
| **Tartratpuffer [mM]** | - | - | - | - | - | - | - | - | **20** | **20** | **20** | **10** |
| pH | 4,5 | 4,75 | 5,0 | 4,75 | 5,0 | 5,0 | 5,0 | 5,0 | 5,0 | 5,5 | 5,0 | 5,0 |
| Biologische Aktivität relativ zur Zeit 0 [80-120%] | + | + | + | + | + | + | + | + | + | + | + | + |

Die oben beschriebenen Versuche zeigen, dass die Stabilität von G-CSF in den erfindungsgemäßen succinatpuffern auch bei pH-Werten, die nahe den physiologischen pH-Werten liegen, über lange Zeit und bei Temperaturen bis mindestens 37°C stabil sind. Die Ergebnisse sind vergleichbar mit Formulierungen in 10 mM Acetat, pH 4,0 und deutlich besser als bei Formulierungen mit 10 mM Phosphatpuffer mit pH 5,0 und 7,5.

### 2. Stabilität von G-CSF-Formulierungen nach mechanischer Belastung

G-CSF-haltige Zusammensetzungen wurden wie in Beispiel 1 beschrieben bei Raumtemperatur hergestellt, indem die Puffersubstanzen Succinat und Tartrat in Form der Dinatriumsalze, gegebenenfalls zusammen mit einem Tensid (Polysorbat-20 oder Polysorbat-80) und einem isotonisierenden Mittel (Mannit oder Sorbit), zunächst in destilliertem und sterilem Wasser gelöst wurden und anschließend der pH-Wert mit Succinat- oder Tartratpuffer auf den gewünschten Wert eingestellt wurde. Im Handel erhältlicher, nicht-glykosylierter rekombinanter humaner G-CSF wurde nach Filtration durch ein Sterilfilter (Porengröße 0,2 µm, Millipore^{®}) zugegeben.

500 µl-Proben der hergestellten Zusammensetzungen wurden in SCF-Fertigspritzen (Becton Dickinson, Grenoble, Frankreich) aufgezogen und auf einem Vortex^{®}-Gerät zur Ausübung von mechanischem Stress 10 sec bei Raumtemperatur geschüttelt.

Die Überprüfung der G-CSF-Zusammensetzungen auf Aggregatbildung nach dem Schütteln erfolgte mittels Größenausschlußchromatographie (SEC) mit einem Agilent Series 1100-Gerät an einer BioSep SEC S2000-Säule (7,8 x 300 mm, 5µm) der Firma Phenomenex. Als Eluens wurde 50 mM Phosphatpuffer, pH 7,0, eingesetzt, der 50 mM NaCl enthielt. Die Analyse erfolgte isokratisch bei einer Durchflussgeschwindigkeit von 0,4 ml/min innerhalb von 45 min und bei einer Säulentemperatur von 20°C. Das Injektionsvolumen lag zwischen 15 und 25 µl, entsprechend einer Proteinmenge von 15 µg. Die Detektionswellenlänge betrug 214 nm und die Größenauswertung erfolgte mittels eines Gelfiltrationsstandards der Firma Biorad (BioRad Art.-Nr. 151-1901) durch Auftragen des Molekulargewichts über dem Elutionsvolumen. Die Aggregatbildung, ausgedrückt in %, gibt den Gehalt an Dimer und höheren Aggregaten von G-CSF in der Probe an, bezogen auf den Anfangsgehalt an monomerem G-CSF vor mechanischer Belastung, und ergibt sich aus den Peakflächen für das Monomer und die auftretenden Aggregate.

Die Probenzusammensetzungen und die Ergebnisse der Tests sind in der nachfolgenden Tabelle 9 angegeben. Die angegebenen Werte stellen Mittelwerte von jeweils drei Versuchen dar.

**Tabelle 9: Aggregatbildung von G-CSF-Formulierungen bei mechanischem Stress**

| | **Formulierung** | **Aggregatbildung [%]** |
|---|---|---|
| 1 | 10 mM Succinatpuffer pH 5,0 0,02 % (w/v) Tween 20 5 % (w/v) D-Sorbit | 0,1 - 0,2 |
| 2 | 10 mM Acetatpuffer pH 4,0 0,004 % (w/v) Tween 80 5 % (w/v) D-Sorbit | 1 - 2 |

Die Ergebnisse zeigen, dass die succinatgepufferte Formulierung selbst bei höheren Temperaturen und einem pH-Wert von 5,0 nach mechanischer Belastung weniger Aggregatbildung aufweist als die acetatgepufferte Formulierung. Die erfindungsgemäßen Zusammensetzungen verhalten sich also gegenüber mechanischem Stress stabiler als herkömmliche Zusammensetzungen.

### 3. Stabilität von G-CSF-Formulierungen nach Einfrieren und Auftauen

### 3.1 Stabilität niedrigkonzentrierter Zusammensetzungen

Es wurden G-CSF-haltige Zusammensetzungen mit einer G-CSF-Konzentration von 0,6 mg/ml hergestellt. Die Herstellung erfolgte wie in Beispiel 2 beschrieben.

Für den Stabilitätstest wurden 500 µl-Proben der hergestellten Zusammensetzungen einem einmaligen Einfrier/Auftauzyklus unterworfen, indem die Proben bei -70°C über Nacht eingefroren und am nächsten Tag bei 20°C aufgetaut wurden. Nach dem Auftauen wurden die Proben sofort wie in Beispiel 2 beschrieben mittels SEC auf ihren Restgehalt an monomerem G-CSF analysiert.

Die Ergebnisse der Tests sind in der nachfolgenden Tabelle 10 angegeben. Die angegebenen Werte stellen Mittelwerte von 3 Tests dar.

**Tabelle 10: Restgehalt an monomerem G-CSF in gepufferten G-CSF-Formulierungen nach Einfrieren und Auftauen**

| | **Formulierung** | **Restgehalt Monomer [%]** |
|---|---|---|
| 1 | 10 mM Succinatpuffer pH 5,0 0,02 % (w/v) Tween 20 5 % (w/v) D-Sorbit | 100 ± 2 |
| 2 | 10 mM Succinatpuffer pH 5,0 | 99 ± 2 |
| 3 | 10 mM Acetatpuffer pH 4,2 0,004 % (w/v) Tween 80 5 % (w/v) D-Sorbit | 50 ± 5 |

### 3.2 Stabilität hochkonzentrierter Zusammensetzungen

G-CSF-haltige Zusammensetzungen wurden entsprechend Beispiel 3.1 hergestellt, außer dass die G-CSF-Konzentration 3,0 mg/ml betrug.

Der Stabilitätstest wurde wie in Beispiel 3.1 beschrieben durchgeführt, außer dass für den Einfrier/Auftauzyklus 1000 µl-Proben der hergestellten Zusammensetzungen verwendet wurden. Nach dem Auftauen wurden die Proben wie in Beispiel 2 beschrieben einer SEC unterworfen und der Gehalt an G-CSF-Dimeren und höheren Aggregaten in der Probe wurde bestimmt und in %, bezogen auf den Anfangsgehalt an monomerem G-CSF in der Probe, angegeben.

Die Ergebnisse der Tests sind in der nachfolgenden Tabelle 11 angegeben. Die angegebenen Werte stellen Mittelwerte von 3 Tests dar.

**Tabelle 11: Aggregatbildung von G-CSF in gepufferten Formulierungen nach einmaligem Einfrieren und Auftauen**

| | **Formulierung** | **Aggregatgehalt [%]** |
|---|---|---|
| 1 | 20 mM Succinatpuffer pH 4,25 | 0,1 -0,13 |
| 2 | 20 mM Succinatpuffer pH 5,0 | 0,1-0,2 |
| 3 | 20 mM Acetatpuffer pH 4,0 | 45 ± 5 |

Die Bildung von Dimeren und höheren Aggregaten wurde auch mittels isoelektrischer Fokussierung verifiziert (Ergebnisse nicht gezeigt).

Die Ergebnisse zeigen, dass succinatgepufferte G-CSF-Formulierungen G-CSF auch bei pH-Werten über 4,0 nach einem Einfrier-/Auftauzyklus noch in nahezu unveränderter Form enthalten, während der Gehalt an monomerem G-CSF in acetatgepufferten Formulierungen stark abnimmt. Die erfindungsgemäßen Zusammensetzungen verhalten sich also gegenüber Einfrier-/Auftauzyklen deutlich stabiler als herkömmliche Zusammensetzungen.

Insgesamt zeigen die obigen Versuche, dass G-CSF in den erfindungsgemäßen Zusammensetzungen auch bei pH-Werten, die nahe den physiologischen pH-Werten liegen, über lange Zeit und bei Temperaturen bis mindestens 37°C stabil sind. Die erfindungsgemäßen Zusammensetzungen eignen sich daher beispielsweise sehr gut für den Einsatz als Injektionslösungen, mit denen sich wegen des physiologischen pH-Werts Hautirritationen verhindern lassen. Die Anwesenheit von Succinat und/oder Tartrat verhindert ferner unerwünschte Reaktionen von G-CSF bei der Rekonstitution des Proteins, beispielsweise beim Auflösen. Die erfindungsgemäßen Zusammensetzungen sind außerdem gegenüber mechanischer Belastung stabil, so dass solche Formulierungen nicht nur problemlos filtriert, in Ampullen abgefüllt oder in Spritzen aufgezogen werden können, sondern sich auch gefahrlos über längere Strecken transportieren lassen. Die erfindungsgemäßen Zusammensetzungen sind auch gegenüber wiederholtem Einfrieren und Auftauen stabil, wodurch die Haltbarkeit der G-CSF-Formulierungen noch weiter verlängert werden kann.

## Patentansprüche

1. Stabile wässrige G-CSF-haltige Zusammensetzung, bestehend aus:
(a) der Puffersubstanz Succinat in Form der freien Säure und/oder eines Salzes davon in einer Konzentration von 0,5 bis 100 mM,
(b) wenigstens einem Tensid,
(c) einem isotonisierenden Mittel, ausgewählt aus Mannit und/oder Sorbit, und
(d) G-CSF.

2. Zusammensetzung nach Anspruch 1, wobei der pH-Wert der Zusammensetzung zwischen 3,5 und 6,0, bevorzugt zwischen 4,0 und 5,8, und besonders bevorzugt zwischen 4,5 und 5,5 liegt.

3. Zusammensetzung nach Anspruch 1 oder 2, wobei die Zusammensetzung mit einem Alkali-, Erdalkali- oder Ammoniumsuccinat gepuffert ist.

4. Zusammensetzung nach Anspruch 3, wobei die Zusammensetzung mit Dinatriumsuccinat gepuffert ist.

5. Zusammensetzung nach einem der Ansprüche 1 bis 4, wobei das Succinat in einer Konzentration von 1 bis 50 mM vorliegt.

6. Zusammensetzung nach einem der Ansprüche 1 bis 5, wobei G-CSF in einer Konzentration von 0,0001 bis 5 mg/ml, insbesondere von 0,0005 bis 4 mg/ml und bevorzugt von 0,01 bis 1,5 mg/ml vorliegt.

7. Zusammensetzung nach einem der Ansprüche 1 bis 6, wobei das Tensid ein nichtionisches Tensid ist, ausgewählt aus der Gruppe bestehend aus Polyoxyethylensorbitanmonolaurat, Polyoxyethylensorbitanmonooleat, Polyoxyethylensorbitanmonostearat, Polyoxyethylensorbitanmonopalmitat, Polyoxyethylensorbitantrioleat und Polyoxyethylensorbitantristearat.

8. Zusammensetzung nach irgendeinem der Ansprüche 1 bis 7, als pharmazeutisches Päparat.

9. Zusammensetzung nach Anspruch 8, wobei das pharmazeutische Präparat eine Injektions- oder Infusionslösung ist.

10. Lyophilisat oder Pulver, umfassend G-CSF sowie Succinat in Form der freien Säure und/oder eines Salzes davon, wenigstens ein Tensid, sowie ein isotonisierendes Mittel, ausgewählt aus Mannit und/oder Sorbit, erhältlich durch Lyophilisieren bzw. Sprühtrocknen einer wässrigen G-CSF-haltigen Zusammensetzung nach einem der Ansprüche 1 bis 9.

11. Lyophilisat oder Pulver nach Anspruch 10, wobei das Salz der Bernsteinsäure ausgewählt ist aus Alkali-, Erdalkali- oder Ammoniumsalzen.

12. Lyophilisat oder Pulver nach Anspruch 11, wobei das Salz der Bernsteinsäure das Di-Natriumsalz ist.

13. Verfahren zur Herstellung wässriger Zusammensetzungen nach irgendeinem der Ansprüche 1 bis 9, welches das Lösen von G-CSF in einem wässrigen Lösungsmittel umfasst, welches als Puffersubstanz Succinat in Form der freien Säure und/oder eines Salzes davon in einer Konzentration von 0,5 bis 100 mM, wenigstens ein Tensid sowie ein isotonisierendes Mittel, ausgewählt aus Mannit und/oder Sorbit, umfasst.

14. Verwendung einer Zusammensetzung nach einem der Ansprüche 1 bis 9 oder eines Lyophilisats oder Pulvers nach einem der Ansprüche 10 bis 12 zur Herstellung pharmazeutischer Präparate, ausgewählt aus Hydrogelen oder Liposomen.

## Claims

1. A stable aqueous G-CSF-containing composition consisting of:
(a) the buffer substance succinate in the form of the free acid and/or a salt thereof in a concentration of from 0.5 to 100 mM,
(b) at least one surfactant,
(c) an isotonizing agent selected from mannitol and/or sorbitol, and
(d) G-CSF.

2. The composition of claim 1, wherein the pH value of the composition is between 3.5 and 6.0, preferably between 4.0 and 5.8, and more preferably between 4.5 and 5.5.

3. The composition of claim 1 or 2, wherein the composition is buffered with an alkali succinate, an alkaline earth succinate or an ammonium succinate.

4. The composition of claim 3, wherein the composition is buffered with disodium succinate.

5. The composition of any of claims 1 to 4, wherein the succinate is present in a concentration of from 1 to 50 mM.

6. The composition of any of claims 1 to 5, wherein G-CSF is present in a concentration of from 0.0001 to 5 mg/ml, in particular of from 0.0005 to 4 mg/ml, and preferably of from 0.01 to 1.5 mg/ml.

7. The composition of any of claims 1 to 6, wherein the surfactant is a non-ionic surfactant selected from the group consisting of polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate and polyoxyethylene sorbitan tristearate.

8. The composition of any of claims 1 to 7 as a pharmaceutical preparation.

9. The composition of claim 8, wherein the pharmaceutical preparation is a solution for injection or infusion.

10. A lyophilisate or powder comprising G-CSF as well as succinate in the form of the free acid and/or a salt thereof, at least one surfactant, as well as an isotonizing agent selected from mannitol and/or sorbitol, obtainable by lyophilization or spray-drying, respectively, of an aqueous G-CSF-containing composition according to any of claims 1 to 9.

11. The lyophilisate or powder of claim 10, wherein the succinic acid salt is selected from alkali, alkaline earth, or ammonium salts.

12. The lyophilisate or powder of claim 11, wherein the the succinic acid salt is the disodium salt.

13. A method for preparing aqueous compositions according to any of claims 1 to 9, comprising dissolving G-CSF in an aqueous solvent comprising, as the buffer substance, succinate in the form of the free acid and/or a salt thereof in a concentration of from 0.5 to 100 mM, at least one surfactant as well as an isotonizing agent selected from mannitol and/or sorbitol.

14. Use of a composition according to any of claims 1 to 9 or of a lyophilisate or powder according to any of claims 10 to 12 for preparing pharmaceutical preparations selected from hydrogels or liposomes.

## Revendications

1. Composition aqueuse stable contenant G-CSF consistant en :
(a) la substance tampon succinate sous forme de l'acide libre et/ou d'un sel de celui-ci en une concentration de 0,5 à 100 mM,
(b) au moins un tensioactif,
(c) un agent d'isotonie choisi parmi le mannitol et/ou le sorbitol, et
(d) G-CSF.

2. Composition selon la revendication 1 où le pH de la composition est situé entre 3,5 et 6,0, de préférence entre 4,0 et 5,8, et de manière particulièrement préférée entre 4,5 et 5,5.

3. Composition selon la revendication 1 ou 2 où la composition est tamponnée avec un succinate alcalin, alcalino-terreux ou d'ammonium.

4. Composition selon la revendication 3 où la composition est tamponnée avec le succinate de disodium.

5. Composition selon l'une des revendications 1 à 4 où le succinate est présent en une concentration de 1 à 50 mM.

6. Composition selon l'une des revendications 1 à 5 où G-CSF est présent en une concentration de 0,0001 à 5 mg/ml, en particulier de 0,0005 à 4 mg/ml et de préférence de 0,01 à 1,5 mg/ml.

7. Composition selon l'une des revendications 1 à 6 où le tensioactif est un tensioactif non ionique choisi dans le groupe consistant en monolaurate de polyoxyéthylènesorbitan, monooléate de polyoxyéthylènesorbitan, monostéarate de polyohyéthylènesorbitan, monopalmitate de polyoxyéthylènesorbitan, trioléate de polyoxyéthylènesorbitan et tristéarate de polyoxyéthylènesorbitan.

8. Composition selon l'une quelconque des revendications 1 à 7 sous forme de préparation pharmaceutique.

9. Composition selon la revendication 8 où la préparation pharmaceutique est une solution pour injection ou perfusion.

10. Lyophilisat ou poudre comprenant G-CSF ainsi que du succinate sous forme de l'acide libre et/ou d'un sel de celui-ci, au moins un tensioactif, ainsi qu'un agent d'isotonie choisi parmi le mannitol et/ou le sorbitol, pouvant être obtenu par lyophilisation ou séchage par pulvérisation d'une composition aqueuse contenant G-CSF selon l'une des revendications 1 à 9.

11. Lyophilisat ou poudre selon la revendication 10 où le sel de l'acide succinique est choisi parmi les sels alcalins, alcalino-terreux et d'ammonium.

12. Lyophilisat ou poudre selon la revendication 11 où le sel de l'acide succinique est le sel de disodium.

13. Procédé pour produire des compositions aqueuses selon l'une quelconque des revendications 1 à 9 qui comprend la dissolution de G-CSF dans un solvant aqueux qui comprend comme substance tampon du succinate sous forme de l'acide libre et/ou d'un sel de celui-ci en une concentration de 0,5 à 100 mM, au moins un tensioactif ainsi qu'un agent d'isotonie choisi parmi le mannitol et/ou le sorbitol.

14. Utilisation d'une composition selon l'une des revendications 1 à 9 ou d'un lyophilisat ou poudre selon l'une des revendications 10 à 12 pour la production de préparations pharmaceutiques choisies parmi les hydrogels et les liposomes.
